# EUROPEAN PATENT APPLICATION

(11) **EP 3 530 264 A1**
(43) Date of publication of application: **28.08.2019**
(21) Application number: 17863022.4
(22) Date of filing: 13.10.2017
(51) Int. Cl.: A61K 8/91, A61K 8/06, A61K 8/73, A61Q 1/02, A61Q 17/04, A61Q 19/00

(54) **OIL-IN-WATER-TYPE EMULSION COMPOSITION**

(30) Priority: 20.10.2016 JP 2016206418
(71) Applicant: Shiseido Company, Ltd., Tokyo 104-0061 (JP)
(72) Inventor: KUBOTA, Shun, Yokohama-shi Kanagawa 224-8558 (JP); WATANABE, Yurika, Yokohama-shi Kanagawa 224-8558 (JP); SUGIYAMA, Yuki, Yokohama-shi Kanagawa 224-8558 (JP)
(74) Representative: Ter Meer Steinmeister & Partner
(86) International application number: PCT/JP2017/037259
(87) International publication number: WO 2018/074379

(57) **Abstract**

The problem to be solved by the present invention is to provide an oil-in-water type emulsion composition that is excellent in feeling in use, particularly excellent in freshness.

An oil-in-water-type emulsion composition comprising: an oil phase; an aqueous phase in which the oil phase is dispersed; a core-corona type microgel in which a hydrophilic group is partially provided on the surface of a hydrophobic gel fine particle as a dispersant that disperses the oil phase in the aqueous phase; and an agar microgel having an average particle size of 10 to 100 µm.

## Description

### RELATED APPLICATION

This application claims the priority of Japanese Patent Application No. 2016-206418 (filed on October 20, 2016), which is incorporated herein by reference.

### FIELD OF THE INVENTION

The present invention relates to an oil-in-water-type emulsion composition and particularly to an oil-in-water-type emulsion composition which provides freshness and excellent durability of makeup.

### BACKGROUND OF THE INVENTION

In conventional emulsification, a surfactant (emulsifier) is necessary to be added to stably disperse a certain liquid in another liquid. Such an emulsifier has an amphipathic molecular structure that compromises specifically polar (hydrophilic) and non-polar (hydrophobic) moieties in the molecule thereof per se, which are spatially apart from each other.

Oil-in-water-type emulsion used in cosmetics allows aqueous components and oil components to be mixed stably based on the emulsifying effect of the added surfactant. That is, finely dispersed liquid drops of an oily (oil) phase are surrounded by shells (micelle) of the emulsifier and the outer phase thereof is an aqueous phase that is the continuous phase, so that it is known as the reason for superior feeling in use along with giving a dewy feeling.

On the other hand, with the increase in the number of consumers who concern more importance of safety, a demand for oil-in-water-type emulsion composition that does not comprise a surfactant that rarely causes irritation to some very sensitive users or comprises a surfactant at an amount such that irritation may not be caused is increasing.

An emulsion prepared by adsorbing powder to the interface without using a surfactant is conventionally known as Pickering emulsion.

A core-corona type microgel characterized in the present invention can be used as a dispersant in Pickering emulsion (powder-emulsification) (Patent Literatures 1 to 11, Non-patent Literatures 1 to 4). However, such an oil-in-water-type emulsion composition obtained with the core-corona type microgel was unsatisfactory in feeling in use, especially in freshness.

### CITATION LIST

### PATENT LITERATURE

[PATENT LITERATURE 1] Japanese Patent No. 2656226
[PATENT LITERATURE 2] Japanese Unexamined Patent Publication (Translation of PCT Application) No. 2001-518111
[PATENT LITERATURE 3] Japanese Unexamined Patent Publication No. 2007-332037
[PATENT LITERATURE 4] Japanese Unexamined Patent Publication No. 2006-36763
[PATENT LITERATURE 5] Japanese Unexamined Patent Publication No. 2008-291026
[PATENT LITERATURE 6] Japanese Unexamined Patent Publication No. H11-158030
[PATENT LITERATURE 7] Japanese Unexamined Patent Publication (Translation of PCT Application) No. 2009-501256
[PATENT LITERATURE 8] Japanese Patent No. 5207424
[PATENT LITERATURE 9] Japanese Patent No. 4577721
[PATENT LITERATURE 10] Japanese Unexamined Patent Publication No. 2006-161026
[PATENT LITERATURE 11] Japanese Unexamined Patent Publication No. 2006-161027

### NON-PATENT LITERATURE

[NON-PATENT LITERATURE 1] B. Blinks et. al, Advances in Colloid and Interface Science, 100-102 (2003).
[NON-PATENT LITERATURE 2] Mukul M, Sharma, et. al, Journal of Colloid and Interface Science, 157, 244-253 (1993).
[NON-PATENT LITERATURE 3] J. Agric. Food Chem., 59, 2636-2645 (2011).
[NON-PATENT LITERATURE 4] J. Colloid Interface Sci., 274, 49 (2004).

### DISCLOSURE OF THE INVENTION

### PROBLEM TO BE SOLVED BY THE INVENTION

The present invention has been made in view of the conventional art, and an object thereof is to provide an oil-in-water-type emulsion composition that is excellent in feeling in use and freshness and also excellent in durability of makeup.

### MEANS TO SOLVE THE PROBLEM

The present inventors have diligently investigated on the afore-mentioned problem, and as a result, they have found that an oil-in-water-type emulsion composition prepared by using a core-corona type microgel as a dispersant and mixing an agar microgel is excellent in feeling in use and freshness and also excellent in durability of makeup, and completed the invention.

That is, an oil-in-water-type emulsion composition of the present invention comprises:
an oil phase;
an aqueous phase in which the oil phase is dispersed;
a core-corona type microgel in which a hydrophilic group is partially provided on the surface of a hydrophobic gel fine particle as a dispersant that disperses the oil phase in the aqueous phase; and
an agar microgel having an average particle size of 10 to 100 µm.

Furthermore, said composition comprises 0.5 to 10% by mass of (acrylates/methoxy PEG methacrylate) crosspolymer as the core-corona type microgel.

Furthermore, said composition comprises 0.5 to 10% by mass of (acrylamide/acarylates/methoxy PEG methacrylate) crosspolymer as the core-corona type microgel.

Furthermore, a powder is dispersed in the internal oil phase.

Furthermore, a blending amount of a non-ionic surfactant is preferably not more than 3% by mass when such a non-ionic surfactant is blended.

Furthermore, a viscosity thereof is preferably not more than 50,000.

Furthermore, a viscosity thereof is more preferably not more than 10,000.

Furthermore, a viscosity thereof is most preferably not more than 5,000.

### EFFECT OF THE INVENTION

The present invention provides an oil-in-water-type emulsified cosmetic excellent in feeling in use, in particular excellent in dewy feeling and durability of makeup, by mixing a core-corona type microgel and an agar microgel.

### BRIEF DESCRIPTION OF THE DRAWINGS

FIG. 1 is an explanation view of d and doused for measuring a swelling ratio of the core-corona type microgel.
FIG. 2 is a graph illustrating the swelling ratio of the core-corona type microgel for each type of oil.

### MODE FOR CARRYING OUT THE INVENTION

The embodiments of the present invention will be described in detail in the following:

### [Aqueous phase]

In the present invention, an agar microgel with an average particle size of 10 to 100 µm is required to be comprised in an aqueous phase

The agar microgel is a microgel having a µm unit, which is prepared by crushing a solidified agar. Any agar can be used without limitation irrespective of the kind of the agar whether the natural product or the commercial product as long as it contains agarose of high gelling ability as the main ingredient. Commercial products of the agar such as Ina-Kanten (tradename) PS-84, Z-10, AX-30, AX-100, AX-200, T-1, S-5, and M-7 (Ina Food Industry Co., Ltd.) may be used preferably. A purified agarose can also be used as the agar.

Production of the microgel will be described in the following.

An agar is dissolved in water or an aqueous component, and then the solution thereof is kept cooled and solidified to form a gel. The dissolution of the agar into water or the aqueous component can be implemented by mixing, heating and the like.

Gelation of the agar is carried out after dissolution by stopping heating and keeping it cooled until the temperature of the agar solution becomes lower than the gelation temperature (solidification temperature).

For the aqueous component, any aqueous component used in the field of cosmetics or pharmaceuticals can be used without limitation. For example, glycols such as 1, 3-butylene glycol and propylene glycol, lower alcohols such as ethanol and propanol, or components generally added as an aqueous component can be used. Specifically, chelating agents such as metaphosphate and edetate, or pH adjusters, and preservatives can be formulated, but not limited thereto.

In conventional oil-in-water-type emulsion compositions, for example, the oil-in-water-type emulsion component tends to cause stickiness when a large amount of a moisturizer such as glycerol is blended to the aqueous phase. On the other hand, in the oil-in-water-type emulsion cosmetic according to the present invention, a moisturizer is blended as an aqueous component of a microgel so that a large amount of said moisturizer can be blended without causing stickiness to the system. Similarly, components that were difficult to blend due to characteristic problems or compatibility with other components, for example, drugs such as arginine, can be blended to the microgel. Accordingly, the oil-in-water-type emulsion composition in the present invention can provide additional functions such as moisturizing property in accordance with the blended components.

Any gel strength of said gel can be acceptable as long as the shape of the gel can be maintained and a microgel can be obtained in the next process. In the present invention, a gel with extremely high gel strength can be used. For instance, a gel with the gel strength of 1,000 g/cm² (by the method stipulated by Japan Agar and Marine Product Processing Cooperatives), or of about 1,000 g/cm² or less can be used. On the other hand, the microgel can be obtained by a gel with extremely weak gel strength of about 30 g/cm². For improving usability, the gel strength is preferably about 100 g/cm².

From the view point of the gel strength, the concentration of the agar in water or the aqueous component is preferably 0.5 to 3%. The oil-in-water-type emulsion component preferably comprises about 20 to 60% by mass of water or the aqueous component as the constituent of the agar microgel and 0.1 to 2% by mass of the agar.

In the present invention, the agar gel is preferably blended with one or more of a hydrophilic thickening compound selected from succinoglycan, carboxymethyl cellulose, xanthan gum, acrylamide or salts thereof. Specifically, succinoglycan or its salt is preferable.

By blending the hydrophilic thickening compound to the agar microgel, specific stickiness or stringiness caused when said compound is directly blended in the aqueous phase, patchiness and the like that occurs upon application can be improved. The gel strength of agar gel is increased so that sedimentation and syneresis of emulsified particles in the composition over time can be suppressed.

It is conventionally known that the hydrophilic thickening compound has low salt-tolerance, and when the hydrophilic thickening compound is blended to a composition with inorganic powder particles, the viscosity of said compound is lowered by salts eluted from the particles. On the other hand, in the present invention, the thickening compound is difficult to be directly affected by eluted salts because the hydrophilic thickening compound is blended in a high salt-tolerant agar gel.

Furthermore, since said hydrophilic thickening compound has no gelling ability, the gel strength of the agar gel can be adjusted by blending the hydrophilic thickening compound. That is, the gel strength is lowered by increasing a blending ratio of the hydrophilic thickening compound.

A blending amount of said hydrophilic thickening compound varies in applications of the oil-in-water-type emulsion composition to which the agar microgel is blended. The blending amount of the hydrophilic thickening compound is preferably 0.5 to 2% by mass with respect to the total constituent component of the agar microgel. When the blending amount is less than 0.5 mass % with respect to the agar microgel, dispersion stability of the composition may not be sufficient. When the blending amount is more than 2% by mass, stickiness may occur.

Next, the agar gel formed as described above is crushed (pulverized) with a homogenizer, a disperser, a mechanical stirrer or the like to obtain a desired microgel.

The degree of crushing can be adjusted depending on the purpose as long as the particle size of the obtained microgel is within the range of the above-described particle size. If more smooth feeling in use is required, the microgel is fully crushed with high-speed stirring to obtain the microgel having a finer particle size. On the other hand, when a tactile impression of the microgel per se is needed, the microgel is less crushed by stirring softly to obtain the microgel having slightly larger particle size.

In the present invention, it is necessary to prepare the microgel with the average particle size of 10 to 100 µm. When the average particle size of the microgel is less than 10 µm, the gelling ability is difficult to be exhibited. When the average particle size of the microgel is more than 100 µm, the particle size difference between the microgel and the emulsion particle becomes too large so that it becomes difficult to disperse the emulsion particle in the solution system by the microgel stably.

A viscosity of microgel obtained as such can be adjusted suitably depending on the purpose of the oil-in-water-type emulsion composition to which said microgel is blended. The preferable viscosity of the microgel is about 2,000 to 1,000,000 mPa·s measured with a B-type viscometer (at the rotation number of 0.6 rpm at 25 °C) when an agar concentration with respect to water or the aqueous component is about 0.5 to 2%.

A blending amount of the agar microgel is preferably 0.05 to 5% by mass, and more preferably 0.1 to 3% by mass. When the blending amount is less than 0.05% by mass, stability of the formulation may deteriorate. When it is more than 5% by mass, freshness may be impaired.

Water, water soluble alcohols, thickeners, etc. commonly used in cosmetics, quasi-drugs, etc. can be blended as aqueous phase components; in addition, appropriate amounts of moisturizers, chelating agents, preservatives, pigments, etc. can also be blended in as desired other than the agar microgel which is the essential component.

The selection of water contained in the oil-in-water emulsified cosmetic of the present invention is not limited in particular; specific examples include purified water, ion-exchanged water, and tap water.

Examples of water soluble alcohols include lower alcohols, polyhydric alcohols, polyhydric alcohol polymers, dihydric alcohol alkyl ethers, dihydric alcohol ether esters, glycerin monoalkyl ethers, sugar alcohols, monosaccharides, oligosaccharides, polysaccharides, and derivatives thereof.

Examples of lower alcohols include ethanol (may be abbreviated as EtOH), propanol, isopropanol, isobutyl alcohol, and t-butyl alcohol.

Examples of polyhydric alcohols include: dihydric alcohols (for example, dipropylene glycol, 1,3-butylene glycol, ethylene glycol, trimethylene glycol, 1,2-butylene glycol, tetramethylene glycol, 2,3-butylene glycol, pentamethylene glycol, 2-butene-1,4-diol, hexylene glycol, and octylene glycol); trihydric alcohols (for example, glycerin and trimethylolpropane); tetrahydric alcohols (for example, diglycerin and pentaerythritol such as 1,2,6-hexanetriol); pentahydric alcohols (for example, xylitol and triglycerin); hexahydric alcohols (for example, sorbitol and mannitol); polyhydric alcohol polymers (for example, diethylene glycol, dipropylene glycol, triethylene glycol, polypropylene glycol, tetraethylene glycol, diglycerin, triglycerin, tetraglycerin, and polyglycerin); dihydric alcohol alkylethers (for example, ethylene glycol monomethyl ether, ethylene glycol monoethyl ether, ethylene glycol monobutyl ether, ethylene glycol monophenyl ether, ethylene glycol monohexyl ether, ethylene glycol mono 2-methyl hexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether, diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether); dihydric alcohol ether esters (for example, ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, and propylene glycol monophenyl ether acetate); glycerin mono alkyl ethers (for example, xylyl alcohol, selachyl alcohol, and batyl alcohol); sugar alcohols (for example, maltotriose, mannitol, sucrose, erythritol, glucose, fructose, starch amylolysis sugar, maltose, and alcohol prepared by the reduction of starch amylolysis sugar); glysolid; tetrahydro furfuryl alcohol; POE-tetrahydro furfuryl alcohol; POP-butyl ether; POP/POE-butyl ether; tripolyoxypropylene glycerin ether; POP-glycerin ether; POP-glycerin ether phosphoric acid; POP/POE-pentane erythritol ether; and polyglycerin.

Examples of monosaccharides include: trioses (for example, D-glyceryl aldehyde and dihydroxyacetone); tetroses (for example, D-etythrose, D-erythrulose, D-threose, and erythritol); pentoses (for example, L-arabinose, D-xylose, L-lyxose, D-arabinose, D-ribose, D-ribulose, D-xylulose, and L-xylulose); hexoses (for example, D-glucose, D-talose, D-psicose, D-galactose, D-fructose, L-galactose, L-mannose, and D-tagatose); heptoses (for example, aldoheptose and heprose); octoses (for example, octurose); deoxysugars (for example, 2-deoxy-D-ribose, 6-deoxy-L-galactose, and 6-deoxy-L-mannose); amino sugars (for example, D-glucosamine, D-galactosamine, sialic acid, amino uronic acid, and muramic acid); and uronic acid (for example, D-glucuronic acid, D-mannuronic acid, L-guluronic acid, D-galacturonic acid, and L-iduronic acid).

Examples of oligosaccharides include sucrose, gentianose, umbelliferose, lactose, planteose, isolignoses, α, α-trehalose, raffinose, lignoses, umbilicine, stachyose and verbascoses.

Examples of polysaccharides include cellulose, quince seed, starch, galactan, dermatan sulfate, glycogen, gum arabic, heparan sulfate, traganth gum, keratan sulfate, chondroitin, xanthan gum, guar gum, dextran, kerato sulfate, locust bean gum, and succinoglucan.

Examples of polyols include polyoxyethylene methyl glucoside (Glucam E-10) and polyoxypropylene methyl glucoside (Glucam P-10).

Examples of natural water-soluble polymers include: plant-type polymers (for example, gum arabic, gum tragacanth, galactan, guar gum, carob gum, karaya gum, carrageenan, pectin, agar, quince seed (Cydonia oblonga), algae colloids (brown algae extract), starches (rice, corn, potato, and wheat), and glycyrrhizic acid); microorganism-type polymers (for example, xanthan gum, dextran, succinoglucan, and pullulan); and animal-type polymers (for example, collagen, casein, albumin, and gelatin).

Examples of semisynthetic water-soluble polymers include: starch-type polymers (for example, carboxymethyl starch and methylhydroxypropyl starch); cellulosic polymers (for example, methyl cellulose, ethyl cellulose, methylhydroxypropyl cellulose, hydroxyethyl cellulose, cellulose sodium sulfate, hydroxypropyl cellulose, carboxymetyl-cellulose, sodium carboxymethyl cellulose, crystal cellulose, and cellulose powder); and alginic acid-type polymers (for example, sodium alginate and propylene glycol alginate).

Examples of synthetic water-soluble polymers include: vinyl polymers (for example, polyvinyl alcohol, polyvinyl methyl ether, polyvinylpyrrolidone, carboxy vinyl polymer); polyoxyethylene-type polymers (for example, polyethylene glycol 20,000, 40,000, 60,000, etc.); acrylic polymers (for example, sodium polyacrylate, polyethylacrylate, and polyacrylamide); polyethyleneimine; and cationic polymers.

Examples of moisturizers include chondroitin sulfate, hyaluronic acid, mucoitin sulfuric acid, charonic acid, atelocollagen, cholesteryl-12-hydroxy stearate, sodium lactate, bile salt, dl-pyrrolidone carboxylic acid salt, short chain soluble collagen, diglycerin (EO)PO adduct, chestnut rose fruit extract, yarrow extract, and sweet clover extract.

Examples of sequestering agents include 1-hydroxy ethane-1,1-diphosphonic acid, 1-hydroxy ethane-1,1-diphosphonic acid tetrasodium salt, disodium edetate, trisodium edetate, tetrasodium edetate, sodium citrate, sodium polyphosphate, sodium metaphosphate, gluconic acid, phosphoric acid, citric acid, ascorbic acid, succinic acid, and trisodium ethylenediaminehydroxyethyl triacetate.

Examples of amino acids include neutral amino acids (for example, threonine and cysteine) and basic amino acids (for example, hydroxylysine). Examples of the amino acid derivatives include sodium acyl sarcosinate (sodium N-lauroyl sarcosinate), acyl glutamate, sodium acyl β-alanine, and glutathione.

Examples of pH adjusters include buffers such as lactic acid-sodium lactate, citric acid-sodium citrate, and succinic acid-sodium succinate.

Examples of thickeners include: xanthan gum, gum arabic, carrageenan, karaya gum, gum tragacanth, carob gum, quince seed (Cydonia oblonga), casein, dextrin, gelatin, sodium pectate, sodium alginate, methyl cellulose, ethyl cellulose, CMC, hydroxy ethyl cellulose, hydroxypropyl cellulose, PVA, PVM, PVP, sodium polyacrylate, carboxy vinyl polymer, locust bean gum, guar gum, tamarind gum, cellulose dialkyl dimethylammonium sulfate, aluminum magnesium silicate, bentonite, hectorite, aluminium magnesium silicate (veegum), laponite, and silicic acid anhydride.

When considering stability, the thickener is preferably blended at 0.01 to 5% by mass.

### [Hydrophobic powder]

In the oil-in-water-type emulsion cosmetic of the present invention, a hydrophobic powder is preferably blended. In the present invention, a core-corona type microgel is used as a dispersant of the oil-in-water-type emulsion cosmetic, so that the use of other surfactants can be suppressed and water-resistance of the hydrophobic powder can be sufficiently exhibited.

The hydrophobic powder used for the present invention is not particularly limited as long as the surface of the powder has hydrophobicity. Examples of the hydrophobic powder include: powders having hydrophobicity in themselves, such as a silicone resin powder and a fluororesin powder; and powders in which surfaces of inorganic powder particles are hydrophobically-treated by a wet method using a solvent, a gas phase method, a mechanochemical method and the like by using silicones such as methyl hydrogen polysiloxane, dimethyl polysiloxane and the like, dextrin fatty acid esters, higher fatty acids, higher alcohols, fatty acid esters, metallic soaps, alkyl ether phosphate, fluorine compounds, or hydrocarbons such as squalene, paraffin and the like. The average particle size of the hydrophobic powder needs to be smaller than that of the emulsion particles that are the oil phase of the present invention. In particular, when the powder is used as an ultraviolet light scattering agent, a hydrophobic powder having an average particle size of 100 nm or less after being crushed in a wet dispersing machine is preferable. Examples of the inorganic powder particles to be hydrophobically-treated includes: titanium dioxide, zinc oxide, talc, mica, sericite, kaolin, titanated mica, black iron oxide, yellow iron oxide, red iron oxide, lapis lazuli, Prussian blue, chromium oxide, chromium hydroxide and the like.

It is known that the remarkable aggregation and coalescence of emulsion particles are likely to occur particularly when hydrophobically-treated particulate titanium dioxide and hydrophobically-treated particulate zinc oxide among these hydrophobic powders are blended together. However, in the oil-in-water emulsion cosmetic according to the present invention, the blending of the microgel as a dispersant enables remarkable improvement in dispersion stability of the powder and emulsification stability. For this reason, in the present invention, when hydrophobically-treated particulate titanium dioxide and hydrophobically-treated particulate zinc oxide are contained as the hydrophobic powder, the composition is particularly highly useful.

It is preferred that the blending amount of the hydrophobically-treated powder in the oil-in-water emulsion cosmetic of the present invention is 0.1 to 35% by mass with respect to the total amount of the composition. When the amount is less than 0.1% by mass, the effect of blending will not be sufficient, and when the amount is more than 35% by mass, emulsification stability may deteriorate.

### [Oil phase]

Examples of the oil phase components include hydrocarbon oils, higher fatty acids, higher alcohols, synthetic ester oils, silicone oils, liquid fats and oils, solid fats and oils, waxes, and perfumes that are commonly used in cosmetics, quasi-drugs, etc., but are not limited thereto.

Examples of the hydrocarbon oils include isododecane, isohexadecane, isoparaffin, liquid petrolatum, ozocerite, squalane, pristane, paraffin, ceresin, squalene, petrolatum, and microcrystallin wax.

Examples of the higher fatty acids include lauric acid, myristic acid, palmitic acid, stearic acid, behenic acid, oleic acid, undecylenic acid, tall oil, isostearic acid, linolic acid, linoleic acid, eicosapentaenoic acid (EPA), and docosahexaenoic acid (DHA).

Examples of the higher alcohols include straight chain alcohols (for example, lauryl alcohol, cetyl alcohol, stearyl alcohol, behenyl alcohol, myristyl alcohol, oleyl alcohol, and cetostearyl alcohol) and branched chain alcohols (for example, monostearyl glycerin ether (batyl alcohol), 2-decyltetradecynol, lanolin alcohol, cholesterol, phytosterol, hexyl dodecanol, isostearyl alcohol, and octyl dodecanol).

Examples of the synthetic ester oils include octyl octanoate, nonyl nonanoate, cetyl octanoate, isopropyl myristate, octyl dodecyl myristate, isopropyl palmitate, butyl stearate, hexyl laurate, myristyl myristate, decyl oleate, hexyl decyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, n-alkylene glycol monoisostearate, neopentyl glycol dicaprate, tripropylene glycol pivalate, diisostearyl malate, glyceryl di-2-heptylundecanoate, glyceryl diisostearate, trimethylolpropane tri-2-ethyl hexanoate, trimethylolpropane triisostearate, pentaerythritol tetra-2-ethylhexanoate, glycerin tri-2-ethylhexanoate, glyceryl trioctanoate, glycerin triisopalmitate, trimethylolpropane triisostearate, cetyl 2-ethyl hexanoate, 2-ethylhexyl palmitate, glycerin trimyristate, tri-2-heptyl undecanoic acid glyceride, castor oil fatty acid methyl ester, oleyl oleate, aceto glyceride, 2-heptylundecyl palmitate, diisobutyl adipate, 2-octyldodecyl N-lauroyl-L-glutamate, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, and triethyl citrate.

Examples of the silicone oils include chain polysiloxanes (for example, dimethylpolysiloxane, methylphenyl polysiloxane, and diphenyl polysiloxane), ring polysiloxanes (for example, octamethylcyclotetrasiloxane, decamethyl cyclopenta siloxane, and dodecamethyl cyclohexa siloxane), silicone resins forming a three-dimensional network structure, silicone rubbers, various modified polysiloxanes (amino-modified polysiloxane, polyether-modified polysiloxane, alkyl-modified polysiloxane, and fluorine-modified polysiloxane), and acryl silicones.

Examples of the liquid fats and oils include avocado oil, tsubaki oil, turtle fatty acid, macademia nut oil, corn oil, mink oil, olive oil, rapeseed oil, egg yolk oil, sesame oil, persic oil, wheat germ oil, sasanquan oil, castor oil, linseed oil, safflower oil, cotton seed oil, perilla oil, soybean oil, peanut oil, tea seed oil, Japanese nutmeg oil, rice bran oil, Chinese gimlet oil, Japanese gimlet oil, jojoba oil, germ oil, and triglycerin.

Examples of the solid fats and oils include cacao butter, coconut oil, horse fat, hydrogenated coconut oil, palm oil, beef tallow, mutton tallow, hydrogenated beef tallow, palm kernel oil, lard, beef bone fat, Japanese core wax nucleus oil, hydrogenated oil, neatsfoot oil, Japanese core wax, and hydrogenated castor oil.

Examples of the waxes include beeswax, candelilla wax, cotton wax, carnauba wax, bayberry wax, tree wax, whale wax, montan wax, bran wax, lanolin, kapok wax, lanolin acetate, liquid lanolin, sugar cane wax, lanolin fatty acid isopropyl ester, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, and POE hydrogenated lanolin ethyl alcohol ether.

Selection of the perfume is not limited in particular; examples include natural perfumes from animals or plants, synthetic perfumes prepared by means of chemical synthesis, and perfume blends thereof. By blending perfume, a cosmetic having a superior durability of fragrance can be obtained.

Specific examples of perfumes include acetivenol, anise aldehyde, anethole, amyl acetate, amyl salicylate, allyl amyl glycolate, allyl caproate, aldehyde C6-20, ambrettolide, ambrettolide, ambroxan, ionone, Iso E Super, eugenol, auranthiol, galaxolide, calone, coumarin, geraniol, geranyl acetate, Sandalore, santalol, sandela, cyclamen aldehyde, cis-3-hexenyl acetate, cis-3-hexenol, citral, citronellyl acetate, citronellol, cineole, dihydromyrcenol, jasmolactone, cinnamic alcohol, cinnamic aldehyde, styralyll acetate, cedryl acetate, cedrol, damascone, damascenone, decalactone, terpinyl acetate, terpineol, tonalid, tonalide, triplal, nerol, bacdanol, vanillin, hydroxycitronellal, phenylethyl acetate, phenylethyl alcohol, hexyl salicylate, vetiveryl acetate, hedione, heliotropin, helional, vertofix, benzyl acetate, benzyl salicylate, benzyl benzoate, pentalide, pentalide, bornyl acetate, myol, musk ketone, methyl anthranilate, methyl dihydrojasmonate, yara yara, lime oxide, linalyl acetate, linalool, limonene, Lyral, lilial, rose oxide, rhodinol, Angelica oil, anise oil, Artemisia vulgaris oil, basil oil, bay oil, Bergamot oil, calamus oil, camphor oil, cananga oil, cardamom oil, cassia oil, cedar wood oil, celery oil, chamomile oil, cinnamon oil, clove oil, coriander oil, cumin oil, dill oil, elemi oil, estragon oil, eucalyptus oil, fennel oil, fenugreek oil, galbanum oil, geranium oil, ginger oil, grapefruit oil, gaiac wood oil, cypress leaf oil, cypress oil, juniper berry oil, lavandin oil, lavender oil, lemon oil, lime oil, mandarin oil, ziram oil, mimosa oil, peppermint oil, spearmint oil, mill oil, myrtle oil, nutmeg oil, oakmoss oil, olibanum oil, opoponax oil, orange oil, parsley oil, patchouli oil, pepper oil, perilla oil, petit grain oil, neroli oil, orange flower, oil, pimento oil, all spice oil, pine oil, rose oil, rosemary oil, clary sage oil, sage oil, sandalwood oil, styrax oil, taget oil, thyme oil, tuberose oil, valerian oil, vetiver oil, violet leaf oil, wintergreen oil, wormwood oil, ylang-ylang oil, yuzu oil, cassie absolute, genet absolute, hyacinth absolute, immortelle absolute, jasmine absolute, jonquil absolute, narcis absolute, rose absolute, violet leaf absolute, and benzoin.

In emulsion compositions obtained by conventional surfactants, the physical properties of the surfactant and the physical properties of the oil component greatly affect emulsifying ability, and the types of the surfactant needed to be changed to change the oil component. However, since the oil-in-water type emulsion cosmetic of the present invention is a Pickering emulsion using the core-corona type microgel as the dispersant, effect on emulsifying ability and stability caused by the types of the oil component is small and therefore a wider range of types of the oil component can be blended.

### [Dispersant]

As a dispersant, it is necessary that a core-corona type microgel in which a hydrophilic group is partially provided on the surface of a hydrophobic gel particulate is comprised.

The core-corona type microgel of the present invention can be obtained by radical polymerizing the following monomers represented by the formulae (1) to (3) or the formulae (1), (2) and (4) under specific conditions.

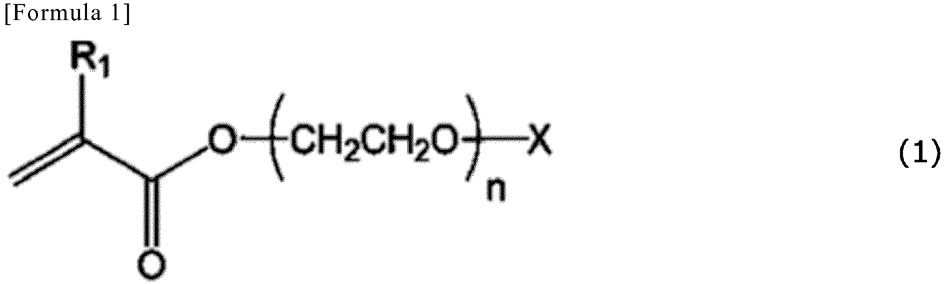

R₁ is an alkyl group having 1 to 3 carbon atoms, n is number of 8 to 300 and X is H or CH₃.

The polyethylene oxide macromonomers of the above formula (1) is preferably an acrylic acid derivative or a methacrylic acid derivative. For the polyethylene oxide macromonomers, commercial products commercially available from Aldrich or BLEMMER® sold by NOF Corporation can be used. Such examples of macromonomers include PME-400, PME-1000, and PME-4000 (n values in formula (1) are 9, 23 and 90, respectively, all products from NOF Corporation), which are methoxy polyethylene glycol monometalate.

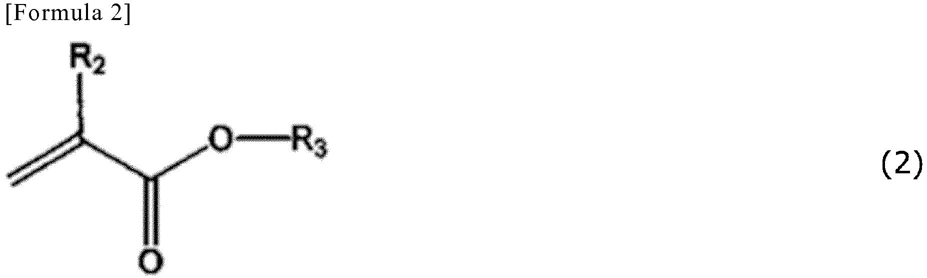

R₂ is an alkyl group having 1 to 3 carbon atoms.

R₃ is an alkyl group having 1 to 12 carbon atoms and R₃, preferably an alkyl group having 1 to 8 carbon atoms.

The hydrophobic monomers of the above formula (2) is preferably an acrylic acid derivative or a methacrylic acid derivative, and methyl acrylate, ethyl acrylate, propyl acrylate, butyl acrylate, pentyl acrylate, hexyl acrylate, heptyl acrylate, octyl acrylate, decyl acrylate, dodecyl acrylate, methyl methacrylate, ethyl methacrylate, propyl methacrylate, butyl methacrylate, pentyl methacrylate, hexyl methacrylate, heptyl methacrylate, octyl methacrylate, decyl methacrylate, dodecyl methacrylate and the like may be used, for example. Among the above, methyl methacrylate, butyl methacrylate, and octyl methacrylate are particularly preferable.

These hydrophobic monomers are commodity raw materials and they can also be obtained easily as general industrial raw materials. Commercial products commercially available from Aldrich or Tokyo Chemical Industry Co., Ltd may be used, for example.

The crosslinking monomer represented by the formula (3) can be obtained as commercial products or industrial raw materials. Such crosslinking monomer is preferably hydrophobic.

The value m is preferably 0 to 2. Specifically, ethylene glycol dimethacrylate (hereinafter referred as to EGDMA) commercially available from Aldrich, BLEMMER® PDE-50 commercially available from NOF CORPORATION and the like are preferably used.

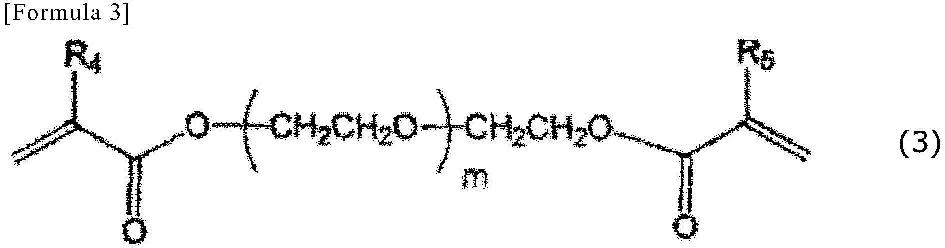

R₄ and R₅ are respectively alkyl groups having 1 to 3 carbon atoms and the value of m is 0 to 2.

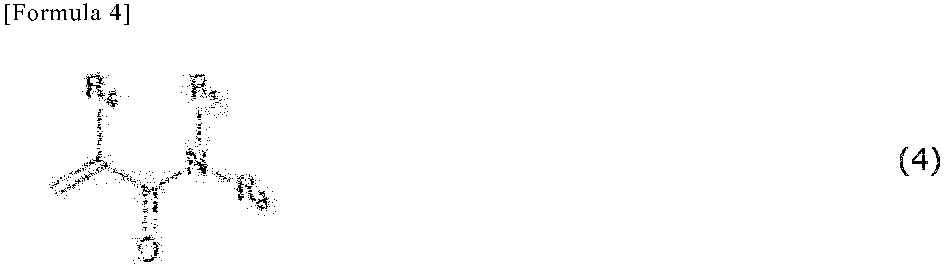

R₆ is H or an alkyl group having 1 to 3 carbon atoms, R₇ and R₈ are substituents including H or an alkyl group having 1 to 12 carbon atoms.

The hydrophobic monomer of the above formula (4) is preferably an acrylamide derivative or a methacrylamide derivative. Examples thereof include: t-butylacrylamide, N,N-dimethylacrylamide, N-[3-(dimethylamino)propyl]acrylamide, t-butylmethacrylamide, octylacrylamide, octyl-methacrylamide, octadecylacrylamide and the like. In the above, t-butylacrylamide, N,N-dimethylacrylamide and N-[3-(dimethylamino) propyl]acrylamide are particularly preferable.

Such hydrophobic monomers can be obtained commercial products or industrial raw materials.

The core-corona type microgel represented by the above formulae (1) to (3) according to the present invention is prepared by radically polymerizing the above monomers under the conditions (A) to (E) shown below:
(A) a molar ratio expressed by a feed molar amount of the polyethylene oxide macromonomer/a feed molar amount of the hydrophobic monomer is 1/1:10/250;
(B) a feed molar amount of the crosslinking monomer is 0.1 to 1.5% by mass with respect to the feed molar amount of the hydrophobic monomer;
(C) the hydrophobic monomer represented by the formula (2) has a monomer composition obtained by mixing one or two or more of a methacrylic acid derivative with an alkyl group having 1 to 8 carbon atoms.
(D) a polymerization solvent is water-organic solvent mixed solvent, and when polyol is used as the organic solvent, polyol is one or two or more selected from dipropylene glycol, 1, 3-butylene glycol and isoprene glycol.
(E) a solvent composition of the water-organic solvent mixed solvent is water/organic solvent = a range of 90/10:10/90 in a mass ratio at 20°C.

In the present invention, "the feed molar amount of the crosslinking monomer with respect to the feed molar amount of the hydrophobic monomer" is defined as crosslink density (% by mass). With respect to the crosslink density of the core-corona type microgel used in the present invention, the feed molar amount of the crosslinking monomer should be 0.1 to 1.5% by mass with respect to the feed molar amount of the hydrophobic monomer under the Condition (B).

### (Condition (A))

For the feed molar amount of the polyethylene oxide macromonomer and the hydrophobic monomer, it can be polymerized when the molar ratio of the polyethylene oxide macromonomer/the hydrophobic monomer is 1/1:10/250. The feed molar amount is preferably 1/1:10/200, and more preferably 1/1:25/100.

When the molar ratio of the hydrophobic monomer is 10 times or less of that of the polyethylene oxide macromonomer, the polymerized polymer becomes water soluble and does not form a core-corona type microgel. When the molar ratio of the hydrophobic monomer is 250 times or more of that of the polyethylene oxide macromonomer, dispersion stabilization by the polyethylene oxide macromonomer becomes insufficient, so that the hydrophobic polymer due to the insoluble hydrophobic monomer may aggregate and precipitate.

### (Condition (B))

By copolymerizing the crosslinking monomer, the microgel can be polymerized of which the hydrophobic polymer of the core part is crosslinked.

When the feed amount of the crosslinking monomer is less than 0.1% by mass of the feed amount of the hydrophobic monomer, the crosslink density becomes low and the microgel may collapse upon swelling. On the other hand, the feed amount is more than 1.5% by mass, aggregation of the microgel occurs and the preferable microgel particles with narrow particle size distribution cannot be polymerized. The feed amount of the crosslinking monomer is preferably 0.2 to 1.0% by mass, more preferably 0.2 to 0.8% by mass, and most preferably 0.2 to 0.5% by mass.

### (Condition (C))

The hydrophobic monomers of the formula (2) preferably have a monomer composition of a mixture of one or two or more methacrylic acid derivatives with an alkyl group having 1 to 8 carbon atoms. When the number of carbon atoms is 0 (when the monomer does not have an end ester bond), the monomers may be too hydrophilic to be emulsion-polymerized adequately. On the other hand, when the number of carbon atoms is 9 or more, it may become a steric hindrance upon the polymerization and the crosslinking structure may not be formed adequately.

### (Condition (D))

It is necessary that the polymerization solvent is a water-organic solvent mixed solvent. As the organic solvent, ethanol, propanol, butanol, polyol and the like can be used. When polyol is used, the preferable polyol is the one that can dissolve a hydrophobic monomer represented by the formula (2) and a crosslinking monomer represented by the formula (3). It is necessary that dipropylene glycol, 1,3-buthylene glycol, and isoprene glycol are used as polyol in the present invention..

Under consideration of that when the polymer solution as-is is used for is the raw material applicable to an industrial production without further purification process such as dialysis, the solvent to be mixed with water should not be an organic solvent such as ethanol, propanol, or butanol, because such solvents may cause irritation when applied on the skin; so that polyol that is generally blendable into cosmetics is preferable.

### (Condition (E))

The solvent composition of the water-organic solvent mixed solvent which is the polymerization solvent is water/organic solvent = a range of 90/10:10/90 in a mass ratio at 20°C. The solvent composition of the water-organic solvent mixed solvent is preferably water/organic solvent = a range of 90/10:10/90 (volume ratio at 20°C), and more preferably water/organic solvent = a range of 80/20:20/80 (volume ratio at 20°C).

For the polymerization solvent, it is necessary to add an organic solvent for homogeneous dissolution of the hydrophobic monomer. The mixing ratio of the organic solvent is 10 to 90 (volume ratio). When the mixing ratio of organic solvent is lower than 10 % by volume, the dissolution of the hydrophobic monomer becomes extremely poor, and polymerization proceeds in the state in which the monomer is as droplets, so that gigantic masses can be formed but no microgel can be formed. When the mixing ratio of the organic solvent exceeds 90 % by volume, an emulsion of the hydrophobic monomer cannot be formed by hydrophobic interaction, so that no emulsion polymerization can proceed and no microgel can be obtained.

The core-corona type microgel represented by the formulae (1), (2) and (4) according to the present invention is prepared by radically polymerizing said monomers under the following conditions (A)' to (E)':
(A)' a molar ratio expressed by a feed molar amount of the polyethylene oxide macromonomer/a feed molar amount of (the acrylate derivative monomer and/or the acrylamide derivative monomer) is 1/1:10/250,
(B)' the macromonomer represented by the formula (1) is an acrylic acid derivative or a methacrylic acid derivative having a polyethylene glycol group with 8 to 200 repeating units,
   the acrylate derivative monomer represented by the formula (2) is an acrylic acid derivative or a methacrylic acid derivative having a substituent comprising an alkyl group having 1 to 12 carbon atoms, and
   the acrylamide derivative monomer represented by the formula (3) is an acrylamide derivative or a methacrylamide derivative having a substituent comprising an alkyl group having 1 to 12 carbon atoms;
(C)' a polymerization solvent is a water-alcohol mixed solvent, and the alcohol is one or two or more selected from ethanol, dipropylene glycol, 1,3-butylene glycol and isoprene glycol; and
(D)' a solvent composition of the water-alcohol mixed solvent is water/alcohol = a range of 90/10 to 10/90 in a mass ratio at 20°C.

Each condition is described in details in the following.

### (Condition (A)')

For the feed molar amount of the polyethylene oxide macromonomer and the hydrophobic monomer (i.e., the sum total of the acrylate derivative monomer and/or acrylamide derivative monomer), it can be polymerized when the molar ratio of the feed molar amount of the polyethylene oxide macromonomer/feed molar amount of the hydrophobic monomers is in the range of 1/1 to 10/250 (molar ratio). The feed molar amount is preferably in a range of 1/1 to 10/200 and more preferably in a range of 1/11 to 25/100.

When the molar amount of the hydrophobic monomer is less than 10 times of that of the polyethylene oxide macromonomer, the polymerized polymer becomes water soluble and does not form a core-corona type microgel. In addition, when the molar amount of the hydrophobic monomer is 250 times or more of that of the polyethylene oxide macromonomer, dispersion stabilization by the polyethylene oxide macromonomer becomes insufficient, so that the hydrophobic polymer due to the insoluble hydrophobic monomer may aggregate and precipitate.

### (Condition (B)')

A condition (B)' has the three conditions (B-1)' to (B-3)' as shown below.

### (B-1)'

The macromonomer represented by the formula (1) is an acrylic acid derivative or a methacrylic acid derivative having a polyethylene-glycol group with 8 to 200 repeating units. When the number of the repeating units is 7 or less, particles that are dispersed stably in a solvent may not be obtained. When the number of the repeating units is more than 200, particles become fine and may be unstable when the composition is blended in a cosmetic.

### (B-2)'

The acrylate derivative monomer represented by the formula (2) is an acrylic acid derivative or a methacrylic acid derivative having a substituent including an alkyl group having 1 to 12 carbon atoms. When the number of carbon atoms is zero (a monomer without a terminal ester bond), the monomer may be too hydrophilic to be emulsion-polymerized adequately. Meanwhile, when the number of carbon atoms is 13 or more, a preferable feeling in use may not be achieved.

### (B-3)'

The acrylamide derivative monomer represented by the formula (3) is an acrylamide derivative or a methacrylamide derivative having substituents including an alkyl group having 1 to 18 carbon atoms.

It is necessary that the hydrophobic monomer according to the present invention has a monomer composition obtained by mixing one or two or more selected from an acrylate derivative monomer represented by the formula (2) and an acrylamide derivative monomer represented by the formula (3).

In the present invention, two types of methacrylate and butyl methacrylate or four types of methacrylate, t-butylacrylamide, N,N-dimethylacrylamide and N-[3-(dimethylamino)propyl]acrylamide are particularly preferably used as the hydrophobic monomers. In the combinations of these hydrophobic monomers, it is preferred to additionally use methoxy polyethylene glycol monometalate as a macromonomer.

In the present invention, the most preferable combinations of a macromonomer and hydrophobic monomers include, but are not limited thereto:
- methoxy polyethylene glycol monometalate having a polyethylene glycol group with 8 to 90, most preferably 15 repeating units, methacrylate and butyl methacrylate;
- methoxy polyethylene glycol monometalate having a polyethylene glycol group with 8 to 200, most preferably 90 repeating units, methyl methacrylate, butyl methacrylate, t-butylacrylamide and N,N-dimethylacrylamide; and N-[3-(dimethylamino)propyl]acrylamide, t-butylmethacrylamide, octylacrylamide, octylmethacrylamide and octadecylacrylamide.

### (Condition (C))'

It is necessary that the polymerization solvent is a water-alcohol mixed solvent. The preferable alcohol is the one that can dissolve the hydrophobic monomer represented by the formulae (2) and (3). Therefore, one or two or more selected from ethanol, dipropylene glycol, 1,3-buthylene glycol, and isoprene glycol are preferable.

### (Condition (D))'

It is preferred that the solvent composition of the water-alcohol mixed solvent, used as the polymerization solvent, is water/alcohol = a range of 90/10 to 10/90, and more preferably a range of 80/20 to 20/80, in the mass ratio at 20°C. When the mixing ratio of alcohol is lower than 10% by volume, dissolution of the hydrophobic monomer becomes extremely poor, so that microparticles may not be formed. When the mixing ratio of alcohol exceeds 90% by volume, an emulsion of the hydrophobic monomer cannot be formed by hydrophobic interaction, so that no emulsion polymerization can proceed and microparticles may not be obtained.

The core-corona type microgel obtained by using polyols according to the present invention does not contain ethanol and the polymerization solvent is the mixture of water and polyol, so that thereby cosmetics, having no skin irritation even for users have sensitive skin, can be easily obtained.

The polymerization initiator used for the polymerization system can be a commercially available polymerization initiator for the ordinary water-soluble thermal radical polymerization. In such a polymerization system, even if such polymerization is implemented without strictly controlling stirring conditions, very narrow particle size distribution of polymerized microgel can be obtained.

Moreover, all microgel prepared by conventional synthetic polymers are based on utilizing a polymer electrolyte such as polyacrylic acid and do not have any acid-resistance and salt-resistance to dispersibility to water. However, when an application of the ingredient to pharmaceutical products and cosmetics is considered, acid-resistance and salt-resistance are very important factors for such an application under physiological conditions. The core-corona type microgel in the present invention is the microgel stabilized with polyethylene oxide chain of non-ionic polymer, so that an acid-resistance and a salt-resistance in dispersibility to water is expected.

The microgel used in the present invention is generated in almost same particle size as the core-corona type polymer microgel with crosslinking at core parts due to the ordering of the hydrophilic macromonomer and the hydrophobic monomer in the solvent.

It is preferred that the amount of the core-corona type microgel of the present invention blended in a cosmetic is 0.5 to 10% by mass (pure content, simply shown in % hereinafter) on the basis of the total amount of the composition. When the amount of the core-corona type microgel blended is less than 0.5% (pure content), it may become difficult to obtain a stable cosmetic. When the amount of the core-corona type microgel blended is more than 10% (pure content), the cosmetic may not be preferable as a composition in view of stability in long-term storage under high temperature conditions, and may be inferior in feeling in use.

The core-corona type microgel of the present invention emulsifies an oil phase component and an aqueous phase component, and forms the oil-in-water type emulsion composition having a structure formed by adsorbing the core-corona type microgel emulsifier on oil drops of the oil phase component dispersed in the aqueous phase component. Therefore, the core-corona type microgel emulsifier of the present invention is excellent in the emulsification capability. When the core-corona type microgel of the present invention is used as an emulsifier, an oil-in-water type emulsion composition that is extremely excellent in emulsification stability can be produced. The core-corona type microgel can obtain sufficient strength even against the behavior of a hydrophobic powder that exists in the oil phases and that has a high specific gravity.

The oil-in-water type emulsion composition of the present invention is produced by mixing and dispersing the core-corona type microgel into water or an aqueous phase component, adding the oil phase component in which the hydrophobic powder is dispersed in a usual method and other components, and stirring the mixture and applying shearing force to emulsify the mixture.

The blending amount of the oil phase components and the water phase components in the oil-in-water type emulsion composition of the present invention are not prescribed in particular. By using (a) the core-corona type microgel as an emulsifier, an oil-in-water type emulsion composition with a wide range of oil phase components/water phase components ratios, ranging from embodiments having smaller oil phase components/water phase components ratios, i.e., smaller blend ratios of the oil phase components (essences, emulsions, etc.) to embodiments having larger blend ratios of the oil phase components (cleansing creams, sunscreens, hair creams, sheet, aerosol, foundation, etc.) can be obtained.

### [Other components]

Other components normally used in external preparations such as cosmetics and quasi-drugs can be blended as necessary in the composition of the present invention as long as the effect of the present invention is not adversely affected; examples of such components include ultraviolet absorbents, powders, organic amines, polymer emulsions, vitamins, and antioxidants.

As water-soluble ultraviolet absorbents, for example, benzophenone ultraviolet ray absorbents such as 2,4-dihydroxy benzophenone, 2,2'-dihydroxy-4-methoxybenzophenone, 2,2'-dihydroxy-4,4'-dimethoxy benzophenone, 2,2',4,4'-tetrahydroxy benzophenone, 2-hydroxy-4-methoxy benzophenone, 2-hydroxy-4-methoxy-4'-methyl benzophenone, 2-hydroxy-4-methoxy benzophenone-5-sulfonate, 4-phenyl benzophenone, 2-ethyl hexyl-4'-phenyl-benzophenone-2-carboxylate, 2-hydroxy-4-n-octoxybenzophenone, 4-hydroxy-3-carboxy benzophenone, phenyl benzimidazole-5-sulfonic acid and its salt; and benzimidazole ultraviolet ray absorbent such as phenylene -bis-benzimidazole-tetrasulfonic acid and its salt, 3-(4'-methyl benzylidene) -d, 1- camphor, 3-benzylidene-d, 1- camphor, urocanic acid and urocanic acid ethyl ester; may be included, but not limited thereto.

As oil-soluble ultraviolet absorbents, for example, benzoic acid ultraviolet absorbents such as paraaminobenzoic acid (PABA), PABA monoglycerin ester, N,N-dipropoxy PABA ethyl ester, N,N-diethoxy PABA ethyl ester, N,N-dimethyl PABA ethyl ester and the N, N-dimethyl PABA butyl ester; anthranilic acid ultraviolet absorbents such as homomenthyl-N-acetyl anthranilate: salicylic acid ultraviolet absorbents such as amyl salicylate, methyl salicylate, homo menthyl salicylate, octyl salicylate, phenyl salicylate, benzyl salicylate and p-isopropanol phenyl salicylate; cinnamic acid ultraviolet absorbents such as an octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate, 2-ethyl hexyl-p-methoxy cinnamate, 2-ethoxyethyl-p-methoxy cinnamate, cyclohexyl-p-methoxy cinnamate, ethyl-α-cyano-β-phenyl cinnamate, 2-ethyl hexyl-α-cyano-β-phenyl cinnamate, glyceryl-mono-2-ethyl hexanoyl-dipara methoxy cinnamate and 3,4,5- tri-methoxy cinnamic acid 3-methyl-4-[methyl bis (trimethylsiloxy) cyril] butyl; 2-phenyl-5-methyl benzoxazole; 2,2'-hydroxy-5-methylphenyl benzotriazole; 2-(2'-hydroxy-5'-t-octyl phenyl) benzotriazole; 2-(2'-hydroxy-5'-methylphenyl) benzotriazole; dibenzalazine; dianisoyl methane; 4-methoxy-4'-t-butyl-dibenzoylmethane, 5-(3,3-dimethyl-2-norbornylidene)-3-pentan-2-on; and octocrylene, may be included, but not limited thereto.

As powder components, for example, inorganic powder (e.g., talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, metal tungustate, magnesium, silica, zeolite, barium sulfate, calcined calcium sulfate (calcined plaster), calcium phosphate, fluorine apatite, hydroxyapatitte, ceramic powder, metallic soap (e.g., zinc myristate, calcium palmitate, aluminum stearate), boron nitride), organic powder (e.g., polyamide resin powder (nylon powder), polyethylene powder, polymethyl methacrylate powder, polystyrene powder, copolymer resin powder of styrene and acrylic acid, benzoguanamine resin powder, poly ethylene tetrafluoride powder, cellulose powder), inorganic white pigments (e.g., titanium dioxide, zinc oxide), inorganic red series color (e.g., iron oxide (red ocher), iron titanate), inorganic brown series color (e.g., γ-iron oxide), inorganic yellow series color (e.g., yellow iron oxide, yellow ocher), inorganic black series color (e.g., black iron oxide, lower titanium oxide), inorganic purple series color (e.g., mango violet, cobalt violet), inorganic green series color (e.g., chromium oxide, chromium hydroxide, cobalt titanate), inorganic blue series color (e.g., sea blue, Berlin blue), pearl color (e.g., titanium oxide coated mica, titanium oxide coated oxybismuth chloride, titanium oxide coated talc, colored titanium oxide coated mica, oxychlorination bismuth, fish scale guanine), metal powdery color (e.g., aluminum powder, copper powder), organic pigments such as zirconium, barium or aluminum lake (e.g., organic pigments such as red organics such as red 201, red 202, red 204, red 205, red 220, red 226, red 228, red 405, orange 203, orange 204, yellow 205, yellow 401, blue 404, red 3, red 104, red 106, red 227, red 230, red 401, red 505, orange 205, yellow 4, yellow 5, yellow 202, yellow 203, green 3 and blue 1, and organics (e.g., red organics such as 201, red 202, red 204, red 205, red 220, red 226, red 228, red 405, orange 203, orange 204, yellow 205, yellow 401 and blue 404, red 3, red 104, red 106, red 227, red 230, red 401, red 505, orange 205, yellow 4, yellow 5, yellow 202, yellow 203, green 3 and blue 1) and natural pigment (e.g., chlorophyll, β-carotene), may be included, but not limited thereto.

As organic amines, for example, monoethanolamine, diethanolamine, triethanolamine, morpholine, tetrakis(2-hydroxypropyl) ethylenediamine, tri-isopropanol amine, 2-amino-2-methyl-1,3-propanediol and 2-amino-2-methyl-1-propanol, may be included, but not limited thereto.

As polymer emulsions, for example, acrylic acid emulsion, polyacrylic acid ethyl emulsion, acrylic resin liquid, polyacrylic alkyl ester emulsion, polyvinyl acetate resin emulsion and natural rubber latex, may be included, but not limited thereto.

As vitamins, for example, vitamins A, B1, B2, B6, C, E and derivatives thereof, and pantothenic acid and derivatives thereof, and biotin, may be included, but not limited thereto.

As other possible components, for example, preservatives (such as methyl paraben, ethyl paraben, butyl paraben and phenoxyethanol), antiphlogistisc (e.g., glycyrrhizic acid derivatives, glycyrrhetinic acid derivatives, salicylic acid derivatives, hinoki thiol, zinc oxide and allantoin), whitening agents (e.g., placenta extract, creeping saxifrage extract and arbutin), various extracts (e.g., phellodendron bark, coptis japonica, lithospermum root, peony, Swertia japonica, birch, sage, loquat, carrot, aloe, mallow, iris, grape, coix seed, sponge gourd, lily, saffron, Cnidium officinale, ginger root, St. John's wort, Ononis, garlic, red pepper, chinpitoki and seaweed), an activator (e.g., royal jelly, photosensitizing dye and cholesterol derivatives), blood circulation accelerators (e.g., nonyl acid vanillylamide, nicotinic acid benzyl ester, nicotinic acid β-butoxyethyl ester, capsaicin, zingerone, cantharides tincture, ichthammol, tannic acid, α-borneol, tocopherol nicotinate, inositol hexanicotinate, cyclandelate, cinnarizine, tolazoline, acetylcholine, verapamil, cepharanthine and γ-oryzanol), antiseborrheic drugs (e.g., sulfur and thianthol) and antiinflammatory agents (e.g., tranexamic acid, thiotaurine, hypotaurine) may be included, but not limited thereto.

As antioxidants, for example, tocopherol, dibutylated hydroxytoluene, butylated hydroxyanisole and gallic acid esters may be included, but not limited thereto.

As antioxidant auxiliary agents, for example, phosphoric acid, citric acid, ascorbic acid, maleic acid, malonic acid, succinic acid, fumaric acid, cephalin, hexametaphosphate, phytic acid and ethylenediaminetetraacetic acid may be included, but not limited thereto.

In the present invention, when safety is considered, it is preferable that antioxidant auxiliary agent is not blended.

Also, not as the emulsifier but for the purpose of controlling tactile feeling in use, controlling drug permeation and such, or improving washing ability when blended into washing agents for skin and hair, surfactants can be blended as the aqueous phase or oil phase component to the oil-in-water emulsified cosmetic of the present invention as long as the effect of the present invention is not adversely affected. Specifically, when more than 3% by mass of the surfactant is blended, freshness of cosmetics may be impaired.

An amphoteric surfactant has at least one cationic functional group and one anionic functional group, is cationic when the solution is acidic and anionic when the solution is alkaline, and has characteristics similar to a nonionic surfactant around the isoelectric point.

Amphoteric surfactants are classified, based on the type of the anionic group, into the carboxylic acid type, the sulfuric ester type, the sulfonic acid type, and the phosphoric ester type. For the present invention, the carboxylic acid type, the sulfuric ester type, and the sulfonic acid type are preferable. The carboxylic acid type is further classified into the amino acid type and the betaine type. Particularly preferable is the betaine type.

Specific examples include: imidazoline type ampholytic surfactants (for example, 2-undecyl-N,N,N-(hydroxyethyl carboxymethyl)-2-imidazoline sodium salt and 2-cocoyl-2-imidazolinium hydroxide-1-carboxyethyloxy 2 sodium salt); and betaine type surfactants (for example, 2-heptadecyl-N-carboxymethyl-N-hydroxyethyl imidazolinium betaine, lauryldimethylaminoacetic acid betaine, alkyl betaine, amide betaine, and sulfobetaine).

Examples of cationic surfactants include quaternary ammonium salts such as cetyltrimethylammonium chloride, stearyltrimethylammonium chloride, behenyltrimehylammonium chloride, behenyldimethylhydroxyethylammonium chloride, stearyldimethylbenzylammonium chloride, and cetyltrimethylammonium methyl sulfate. Other examples include amide amine compounds such as stearic diethylaminoethylamide, stearic dimethylaminoethylamide, palmitic diethylaminoethylamide, palmitic dimethylaminoethylamide, myristic diethylaminoethylamide, myristic dimethylaminoethylamide, behenic diethylaminoethylamide, behenic dimethylaminoethylamide, stearic di ethylaminopropylamide, stearic dimethylaminopropylamide, palmitic diethylaminopropylamide, palmitic dimethylaminopropylamide, myristic diethylaminopropylamide, myristic dimethylaminopropylamide, behenic diethylaminopropylamide, and behenic dimethylaminopropylamide.

Anionic surfactants are classified into the carboxylate type such as fatty acid soaps, N-acyl glutamates, and alkyl ether acetates, the sulfonic acid type such as □-olefin sulfonates, alkane sulfonates, and alkylbenzene sulfonates, the sulfuric ester type such as higher alcohol sulfuric ester salts, and phosphoric ester salts. Preferable are the carboxylate type, the sulfonic acid type, and the sulfuric ester salt type; particularly preferable is the sulfuric ester salt type.

Specific examples include fatty acid soaps (for example, sodium laurate and sodium palmitate); higher alkyl sulfuric acid ester salts (for example, sodium lauryl sulfate and potassium lauryl sulfate); alkyl ether sulfuric acid ester salts (for example, POE-triethanolamine lauryl sulfate and sodium POE-lauryl sulfate); N-acyl sarcosinic acids (for example, sodium lauroyl sarcosinate); higher fatty acid amide sulfonic acid salts (for example, sodium N-myristoyl N-methyl taurate, sodium cocoyl methyl taurate, and sodium laurylmethyl taurate); phosphoric ester salts (for example, sodium POE-oleyl ether phosphate and POE stearyl ether phosphoric acid); sulfosuccinates (for example sodium di-2-ethylhexylsulfosuccinate, sodium monolauroyl monoethanol amide polyoxyethylene sulfosuccinate, and sodium lauryl polypropylene glycol sulfosuccinate); alkyl benzene sulfonates (for example, sodium linear dodecyl benzene sulfonate, triethanolamine linear dodecyl benzene sulfonate, and linear dodecyl benzene sulfonic acid); higher fatty acid ester sulfates (for example, hydrogenated coconut oil aliphatic acid glycerin sodium sulfate); N-acyl glutamates (for example, mono sodium N-lauroylglutamate, disodium N-stearoylglutamate, and sodium N-myristoyl-L-glutamate); sulfated oils (for example, turkey red oil); POE-alkyl ether carboxylic acid; POE-alkyl aryl ether carboxylate; α-olefin sulfonate; higher fatty acid ester sulfonates; sec-alcohol sulfates; higher fatty acid alkyl amide sulfates; sodium lauroyl monoethanolamine succinates; ditriethanolamine N-palmitoylaspartate; and sodium caseinate.

A nonionic surfactant is a surfactant that is not ionized to have an electric charge in an aqueous solution. For the hydrophobic group, a type that uses alkyls and a type that uses dimethyl silicone are known among others. Specific examples of the former include glycerol fatty acid esters, ethylene oxide derivatives of glycerol fatty acid esters, polyglycerol fatty acid esters, propylene glycol fatty acid esters, ethylene oxide derivatives of propylene glycol fatty acid esters, polyethylene glycol fatty acid esters, polyethylene glycol alkyl ethers, polyethylene glycol alkyl phenyl ethers, polyethylene glycol castor oil derivatives, and polyethylene glycol hydrogenated castor oil derivatives. Examples of the latter include polyether-modified silicone and polyglycerin-modified silicone. Preferable is the type that uses alkyl for the hydrophobic group.

Specific examples of lipophilic nonionic surfactants include sorbitan fatty acid esters (for example, sorbitan mono oleate, sorbitan mono isostearate, sorbitan mono laurate, sorbitan mono palmitate, sorbitan mono stearate, sorbitan sesquioleate, sorbitan trioleate, diglycerol sorbitan penta-2-ethylhexylate, diglycerol sorbitan tetra-2-ethylhexylate); glycerin polyglycerin aliphatic acids (for example, mono cottonseed oil fatty acid glycerin, glyceryl monoerucate, glycerin sesquioleate, glyceryl monostearate, α, α'-glycerin oleate pyroglutamate, monostearate glycerin malic acid); propylene glycol fatty acid esters (for example, propylene glycol monostearate); hydrogenated castor oil derivatives; and glycerin alkylethers.

Examples of hydrophilic nonionic surfactants include POE-sorbitan fatty acid esters (for example, POE-sorbitan monooleate, POE-sorbitan monostearate, POE-sorbitan monooleate, and POE-sorbitan tetraoleate); POE sorbitol fatty acid esters (for example, POE sorbitol monolaurate, POE-sorbitol monooleate, POE-sorbitolpentaoleate, and POE-sorbitol monostearate); POE-glycerin fatty acid esters (for example, POE-monooleates such as POE-glycerin monostearate, POE-glycerin monoisostearate, and POE-glycerin triisostearate); POE-fatty acid esters (for example, POE-distearate, POE-monodioleate, and ethylene glycol distearate); POE-alkylethers (for example, POE-lauryl ether, POE-oleyl ether, POE-stearyl ether, POE-behenyl ether, POE-2-octyl dodecyl ether, and POE-cholestanol ether); pluaronics (for example, pluaronic); POE.cndot. POP-alkylethers (for example, POE.cndot. POP-cetyl ether, POE.cndot. POP-2-decyl tetradecyl ether, POE.cndot. POP-monobutyl ether, POE.cndot. POP-lanolin hydrate, and POE.cndot. POP-glycerin ether); tetra POE.cndot. tetra POP-ethylenediamino condensates (for example, tetronic); POE-castor oil hydrogenated castor oil derivatives (for example, POE-castor oil, POE-hydrogenated castor oil, POE-hydrogenated castor oil monoisostearate, POE-hydrogenated castor oil triisostearate, POE-hydrogenated castor oil monopyroglutamic monoisostearic diester, and POE-hydrogenated castor oil maleic acid); POE-beeswax-lanolin derivatives (for example, POE-sorbitol beeswax); alkanol amides (for example, palm oil fatty acid diethanol amide, laurate monoethanolamide, and fatty acid isopropanol amide); POE-propylene glycol fatty acid esters; POE-alkylamines; POE-fatty acid amides; sucrose fatty acid esters; alkyl ethoxydimethylamine oxides; and trioleyl phosphoric acid.

A viscosity of an oil-in-water-type emulsion composition of the present invention is preferably 10,000 mPa·s or less. Furthermore, the viscosity thereof is more preferably 5,000 mPa·s or less. When the viscosity exceeds 10,000 mPa·s, spreadability may become heavy and freshness of the cosmetic may deteriorate.

Application of the oil-in-water-type emulsion composition of the present invention is not limited. The oil-in-water-type emulsion composition can be commercialized as skin cosmetics such as foundations and sunscreen cosmetics, hair cosmetics, skin external agent and the like.

### EXAMPLES

The present invention will be described with reference to the following examples, but the present invention is not limited thereto. The blending amounts are expressed with "% by mass" unless otherwise specified. EtOH, DPG and BG described in the tables are abbreviations for ethanol, dipropylene glycol and 1,3-butylene glycol, respectively.

### [Example 2: Production example of a core-corona type microgel]

Macro monomer and hydrophobic monomer described in Table 1 were radically polymerized under polymerization conditions shown in Tables 1 and 2 in accordance with the following production method (Technique 1). The appearance of the obtained copolymer dispersion was evaluated visually, and the sizes of particles and the degree of dispersion of the copolymers were evaluated in accordance with Technique 2. Results are shown in Table 3.

### <Technique 1: Production method of a core-corona type microparticle >

Polyethyleneoxide macro monomer and hydrophobic monomer were added into 90g of water-alcohol mixed solvent in a three-neck flask equipped with a reflux tube and a nitrogen feeding tube. After sufficient dissolution or dispersion, dissolved oxygen was removed by nitrogen substitution for 20 minutes. Then, 1 mol% of the polymerization initiator, 2,2'-azobis(2-methylpropionamidine) dihydrochloride, with respect to the total amount of monomers, was dissolved in a small amount of water and added, and further dissolution or dispersion was carried out. The uniformly dissolved or dispersed polymerization solution was put through nitrogen substitution for 20 minutes to remove dissolved oxygen, followed by 8 hours of polymerization with stirring by means of a magnetic stirrer while the temperature was maintained at 65 to 70°C in an oil bath. After the completion of polymerization, the polymer solution was returned to room temperature; thus a core-corona type microparticle dispersion was obtained.

As a polyethylene oxide macro-monomer, Blemmer PME-4000 (produced by NOF CORPORATION) was used. As hydrophobic monomers, methyl methacrylate (MMA), butyl methacrylate (n-BMA), t-butylacrylamide (t-BAA), N,N-dimethylacrylamide (DMAA) and N-[3-(dimethylamino)propyl]acrylamide (DMAPA) were used.

### <Technique 2: Method for measuring the particle size and the degree of dispersion >

The particle size of copolymers was measured using a Zetasizer manufactured by Malvern Instruments Ltd. Measurement samples of the microparticle dispersion liquid with the microparticle concentration of about 0.1% were prepared by dilution with water. After removing dust with a 0.45 µm filter, the scattering intensity at 25°C was measured at the scattering angle of 173° (back-scattered light), the average particle size and the degree of dispersion were calculated with analysis software installed on the measurement apparatus. The particle size was analyzed by the cumulant analysis method. The degree of dispersion is a normalized value of the second-order cumulant value obtained by the cumulant analysis. The degree of dispersion is a commonly used parameter, and the automatic analysis is possible by using a commercial dynamic light scattering measurement apparatus. For the viscosity of the solvent, which was necessary for the particle size analysis, the viscosity of pure water at 25°C, i.e., 0.89 mPa·s, was used.

**[Table-1]**

| | Macromonomer | | Hydrophobic monomer | | | | | Polymerization solvent | | |
|---|---|---|---|---|---|---|---|---|---|---|
| | Methoxy PEG-4000 | Methoxy PEG-1000 | MMA | *n*-BMA | t-BAA | DMAA | DMAPA | Water | Alcohol | Amounts of alcohol |
| | Formula (1) | | Formula (2) | Formula (2) | Formula (3) | Formula (3) | Formula (3) | | | |
| Production example 1 | 4.07 | | 2.45 | 3.48 | | | | 54 | EtOH | 36 |
| Production example 2 | 3.95 | | | | 6.05 | | | 73.8 | EtOH | 16.2 |
| Production example 3 | 3.84 | | | | 4.71 | | 1.45 | 73.8 | EtOH | 16.2 |
| Production example 4 | 3.89 | | | | 4.77 | 0.23 | 1.10 | 73.8 | EtOH | 16.2 |
| Production example 5 | 3.90 | | 0.09 | | 4.67 | 0.23 | 1.10 | 73.8 | EtOH | 16.2 |
| Production example 6 | 4.06 | | 2.40 | 3.41 | 0.06 | | 0.08 | 54 | EtOH | 36 |
| Production example 7 | 2.46 | | 0.30 | | 6.03 | 0.29 | 0.93 | 70.2 | EtOH | 19.8 |
| Production example 8 | 2.46 | | 0.30 | | 6.03 | 0.29 | 0.93 | 58.5 | DPG | 31.5 |
| Production example 9 | | 1.59 | 2.07 | | 5.26 | | 1.08 | 58.5 | BG | 31.5 |
| Production example 10 | 3.03 | | 0.27 | | 5.57 | 0.27 | 0.86 | 70.2 | EtOH | 19.8 |

| | | | | | | | | | | |
|---|---|---|---|---|---|---|---|---|---|---|
| 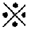 All units in Table-1 are g (gram). | | | | | | | | | | |

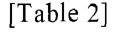

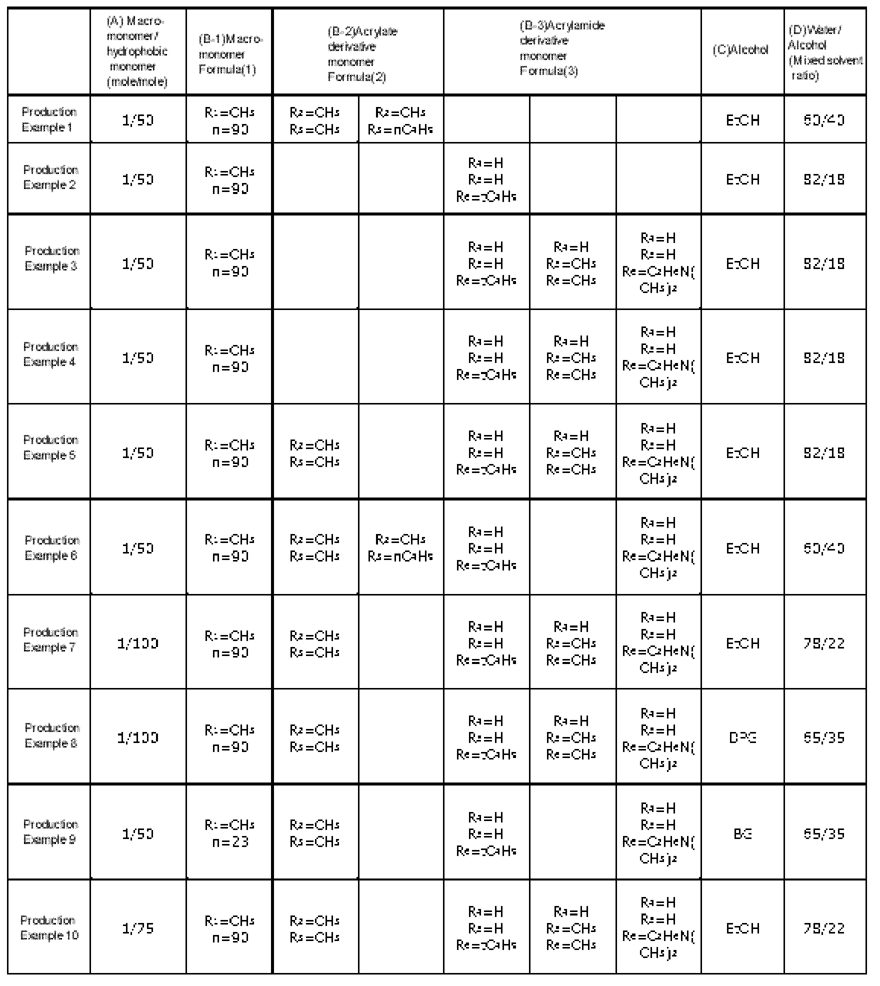

**[Table 3]**

| Production Example | Appearance | Core-corona type microparticle concentration (wt%) | Alcohol type·alcohol concentration (wt%) | Water concentration (wt%) | Particle size (nm) | Degree of dispersion |
|---|---|---|---|---|---|---|
| Production example 1 | white cloudy liquid | 10 | Ethanol·36 | 90 | 187.0 | 0.017 |
| Production example 2 | white cloudy liquid | 10 | Ethanol·16.2 | 90 | 153.6 | 0.019 |
| Production example 3 | white cloudy liquid | 10 | Ethanol· 16.2 | 90 | 191.2 | 0.01 |
| Production example 4 | white cloudy liquid | 10 | Ethanol ·16.2 | 90 | 167.2 | 0.002 |
| Production example 5 | white cloudy liquid | 10 | Ethanol· 16.2 | 90 | 166.5 | 0.028 |
| Production example 6 | white cloudy liquid | 10 | Ethanol·36 | 90 | 210.3 | 0.018 |
| Production example 7 | white cloudy liquid | 10 | Ethanol· 19.8 | 90 | 250.0 | 0.003 |
| Production example 8 | white cloudy liquid | 10 | DPG·31.5 | 90 | 174.6 | 0.014 |
| Production example 9 | white cloudy liquid | 10 | BG·31.5 | 90 | 249.9 | 0.149 |
| Production example 10 | white cloudy liquid | 10 | Ethanol· 19.8 | 90 | 197.1 | 0.006 |

As shown in Table 3, in Production Examples 1 to 10 that polymerized methoxy polyethylene glycol monometalate (macromonomer) and one or two or more of a hydrophobic monomer selected from methyl methacrylate, butyl methacrylate, t-butylacrylamide, N,N-dimethylacrylamide, and N-[3-(dimethylamino)propyl]acrylamide having a substituent comprising an alkyl group having 1 to 4 carbon atoms in a water-ethanol mixed solvent (water: ethanol = 40 to 60:18 to 82) under the condition that the value of "the feed molar amount of a macromonomer/the feed molar amount of a hydrophobic monomer" was 1:50 to 100, white cloudy solution-like dispersions were obtained, and it was possible to evaluate the particle sizes and the degrees of dispersion. That is, the formation of particulate polymers (core-corona type microparticles) was confirmed. It was found that in the core-corona type microparticles of Production examples 1 to 10, the particle sizes were 153.6 to 250.0 nm, the degrees of dispersion were 0.002 to 0.149 and the particle sizes were uniform.

Therefore, the core-corona type microgel in a uniform particle size can be obtained by radically polymerizing a polyethylene oxide macromonomer represented by the formula (1), and one or more of a hydrophobic monomer selected from an acrylate derivative monomer represented by the formula (2) and an acrylamide derivative monomer represented by the formula (4) under the following conditions (A)' to (D)';
(A)' a molar ratio expressed by a feed molar amount of the polyethylene oxide macromonomer/a feed molar amount of (the acrylate derivative monomer and/or the acrylamide derivative monomer) is 1/1 to 10/250,
(B)' the macromonomer represented by the formula (1) is an acrylic acid derivative or a methacrylic acid derivative having a polyethylene glycol group with 8 to 200 repeating units,
   the acrylate derivative monomer represented by the formula (2) is an acrylic acid derivative or a methacrylic acid derivative having a substituent comprising an alkyl group having 1 to 12 carbon atoms, and
   the acrylamide derivative monomer represented by the formula (3) is an acrylamide derivative or a methacrylamide derivative having a substituent comprising an alkyl group having 1 to 12 carbon atoms;
(C)' a polymerization solvent is a water-alcohol mixed solvent, and the alcohol is one or two or more selected from ethanol, dipropylene glycol, 1,3-butylene glycol and isoprene glycol; and
(D)' a solvent composition of the water-alcohol mixed solvent is water/alcohol = a range of 90/10 to 10/90 in a mass ratio at 20°C.

Next, the inventors investigated on the powder-in-oil-in-water type cosmetics of which the microgel obtained in the above-described production examples are blended, mainly in terms of the blending forms of the hydrophobic powder, stability of the formulation thereof and water-resistance upon application. The results are shown in Tables 4 and 5.

The evaluation was implemented as follows:

### Evaluation (1): Feeling in Use

The feeling in use ("lightness in spreadability" and "freshness") when the sample was applied to skin was evaluated by 10 professional panelists based on the following criteria:
A: 7 or more experts out of 10 experts answered "good" or "really felt".
B: 5 or more experts out of 10 experts answered "good" or "really felt".
C: 3 or more experts out of 10 experts answered "good" or "really felt".
D: 2 experts or less out of 10 experts answered "good" or "really felt".

### Evaluation (2): Freshness

Freshness on skin when the sample was applied to skin was evaluated by 10 professional panelists based on the following criteria:
A: 7 or more experts out of 10 experts answered "good" or "really felt".
B: 5 or more experts out of 10 experts answered "good" or "really felt".
C: 3 or more experts out of 10 experts answered "good" or "really felt".
D: 2 experts or less out of 10 experts answered "good" or "really felt".

### Evaluation (3): Rolling stability

Sample was put into 50 ml sample tube (diameter: 3 cm) and the rolling test was implemented at the rotating speed 45 rpm for four hours at room temperature. Then, aggregation of powders was observed visually.
A: No color stripe was observed visually.
B: A color stripe was slightly observed visually.
C: A color stripe was observed visually.
D: A considerable number of color stripes was observed visually.

### Evaluation (3): Durability of makeup

Skin condition in ten hours after the sample was applied to skin was evaluated by 10 professional panelists based on the following criteria:
A: No shininess of skin (irregular sebum) was observed visually.
B: Slight shininess of skin (irregular sebum) was observed visually.
C: Shininess of skin (irregular sebum) was observed visually.
D: A considerable amount of shininess of skin (irregular sebum) was observed visually.

**[Table 4-1]**

| | Raw materials | Example 4-1 | Example 4-2 | Example 4-3 | Example 4-4 | Example 4-5 | Example 4-6 | Example 4-7 |
|---|---|---|---|---|---|---|---|---|
| Core-corona type microgel | (Acrylates methoxy PEG-90 methacrylate) crosspolymer | 1 | 0.5 | 3 | 1 | 1 | 0.5 | 10 |
| Agar microgel | Agar | 0.5 | 0.5 | 0.5 | 0.05 | 5 | 5 | 5 |
| Activator | PEG-60 hydrogenated castor oil | - | 0.5 | 0.5 | - | - | - | - |
| Alcohol | Ethanol | 2 | 2 | 2 | 2 | 2 | 2 | 2 |
| Moisturizer | Dipropylene glycol | 10 | 10 | 10 | 10 | 10 | 10 | 10 |
| | Glycerin | 5 | 5 | 5 | 5 | 5 | 5 | 5 |
| Preservative | Phenoxyethanol | 0.5 | 0.5 | 0.5 | 0.5 | 0.5 | 0.5 | 0.5 |
| Silicone oil | Decamethyl cyclopenta siloxane | 17 | 17 | 17 | 17 | 17 | 17 | 17 |
| Ultraviolet absorbent | Octylmethoxy cinnamate | 3 | 3 | 3 | 3 | 3 | 3 | 3 |
| Water | | Balance | Balance | Balance | Balance | Balance | Balance | Balance |
| Total amount | | 100 | 100 | 100 | 100 | 100 | 100 | 100 |
| Stability | | A | A | A | B | A | B | A |
| Feeling in use | | A | A | B | A | B | A | B |
| Durability of makeup | | A | B | A | A | A | B | A |

| | | | | | | | | |
|---|---|---|---|---|---|---|---|---|
| * Silicone treatment | | | | | | | | |

**[Table 4-2]**

| | Raw materials | Example 4-8 | Example 4-9 | Example 4-10 |
|---|---|---|---|---|
| Core-corona type microgel | (Acrylates methoxy PEG-90 methacrylate) crosspolymer | 3 | 3 | 3 |
| Agar microgel | Agar | 0.3 | 0.3 | 0.3 |
| Activator | PEG-60 hydrogenated castor oil | - | - | - |
| Alcohol | Ethanol | 2 | 2 | 2 |
| Moisturizer | Dipropylene glycol | 10 | 10 | 10 |
| | Glycerin | 5 | 5 | 5 |
| Preservative | Phenoxyethanol | 0.5 | 0.5 | 0.5 |
| Silicone oil | Decamethyl cyclopenta siloxane | 17 | 17 | 17 |
| Neutralizer | Triethanolamine | | | 0.5 |
| Ultraviolet absorbent | Octylmethoxy cinnamate | 7 | | 3 |
| | Ethylhexyl Triazone | 1 | | |
| | 2-Hydroxy-4-methoxybenzophenone | 1 | | |
| | Bis-Ethylhexyloxyphenol Methoxyphenyl Triazine | 1 | 1 | |
| | Diethylamino Hydroxybenzoyl Hexyl Benzoate | 2 | 2 | |
| | Octocrylene | | 3 | |
| | Phenylbenzimidazole Sulfonic Acid | | | 1 |
| Water | | Balance | Balance | Balance |
| Total amount | | 100 | 100 | 100 |
| Stability | | B | B | B |
| Feeling in use | | B | B | B |
| Durability of makeup | | A | A | A |

**[Table 4-3]**

| | Raw materials | Comparative Example 4-1 | Comparative Example 4-2 | Comparative Example 4-3 | Comparative Example 4-4 | Comparative Example 4-5 | Comparative Example 4-6 |
|---|---|---|---|---|---|---|---|
| Core-corona type microgel | (Acrylates methoxy PEG-90 methacrylate) crosspolymer | - | 1 | 1 | 3.5 | 0.25 | 12 |
| Agar micro gel | Agar | 0.5 | - | 5.5 | 0.3 | 0.3 | 0.3 |
| Activator | PEG-60 hydrogenated castor oil | 1 | - | - | 0.5 | - | - |
| Alcohol | Ethanol | 2 | 2 | 2 | 2 | 2 | 2 |
| Moisturizer | Dipropylene glycol | 10 | 10 | 10 | 10 | 10 | 10 |
| | Glycerin | 5 | 5 | 5 | 5 | 5 | 5 |
| Preservative | Phenoxyethanol | 0.5 | 0.5 | 0.5 | 0.5 | 0.5 | 0.5 |
| Silicone oil | Decamethvl cyclopenta siloxane | 17 | 17 | 17 | 17 | 17 | 17 |
| Ultraviolet absorbent | Octylmethoxy cinnamate | 3 | 3 | 3 | 3 | 3 | 3 |
| Water | | Balance | Balance | Balance | Balance | Balance | Balance |
| Total amount | | 100 | 100 | 100 | 100 | 100 | 100 |
| Stability | | A | C | A | A | C | A |
| Feeling in use | | D | A | C | C | A | C |
| Durability of makeup | | D | A | A | A | C | A |

| | | | | | | | |
|---|---|---|---|---|---|---|---|
| * Silicone treatment | | | | | | | |

It is obvious from Test examples 4-1, 4-4 and 4-5 and Comparison examples 4-2 and 4-3 in Table 4 that when the blending amount of the agar microgel is too small, stability deteriorates and when too much, freshness deteriorates. Moreover, it is obvious from Test examples 4-1, 4-6 and 4-7 and Comparison examples 4-5 and 4-6 that when the amount of the core-corona type microgel is too small, emulsification becomes poor and stability cannot maintained and when too much, freshness deteriorates. It is obvious from Test examples 4-1, 4-2 and 4-3 and Comparison example 4-4 that emulsifiers can be blended within the range that does not affect usage of the cosmetic.

Furthermore, it is obvious from Test examples 4-8 to 4-10 that even when ultraviolet absorbers other than octyl methoxycinnamate are used, the effect of the present invention was not impaired..

**[Table 5-1]**

| | Raw materials | Example 5-1 | Example 5-2 | Example 5-3 | Example 5-4 | Example 5-5 | Example 5-6 | Example 5-7 | Example 5-8 |
|---|---|---|---|---|---|---|---|---|---|
| Core-coronatype microgel | (Acrylates methoxy PEG-90 methacrylate) crosspolymer | 1 | 0.5 | 3 | 1 | 1 | 0.5 | 10 | 3 |
| Agar micro gel | Agar | 0.3 | 0.3 | 0.3 | 0.05 | 5 | 5 | 5 | 0.3 |
| Activator | PEG-60 hydrogmated castor oil | - | 0.5 | 0.5 | - | - | - | - | 0.5 |
| Alcohol | Ethanol | 1.5 | 1.5 | 1.5 | 1.5 | 1.5 | 1.5 | 1.5 | 1.5 |
| Moisturizer | Dipropylene glycol | 9 | 9 | 9 | 9 | 9 | 9 | 9 | 9 |
| | Glycerin | 2 | 2 | 2 | 2 | 2 | 2 | 2 | 2 |
| Preservative | Phenoxyethanol | 0.5 | 0.5 | 0.5 | 0.5 | 0.5 | 0.5 | 0.5 | 0.5 |
| Silicone oil | Decamethyl cyclopenta siloxane | 16 | 16 | 16 | 16 | 16 | 16 | 16 | 16 |
| Ultraviolet absorbent | Octylmethoxy cinnamate | 4 | 4 | 4 | 4 | 4 | 4 | 4 | 4 |
| Dispersant | Bis-Butyldimethicone Polyglyceryl-3 | 1.5 | 1.5 | 1.5 | 1.5 | 1.5 | 1.5 | 1.5 | 1.5 |
| | Isostearic acid | 1 | 1 | 1 | 1 | 1 | 1 | 1 | 1 |
| Hydrophobic powder | Hydrophobically-treated* pigment grade titanium oxide | 8 | 8 | 8 | 8 | 8 | 8 | 8 | |
| | Hydrophobically-treated* particulate titanium oxide | 2.5 | 2.5 | 2.5 | 2.5 | 2.5 | 2.5 | 2.5 | 2 |
| | Hydrophobically-treated* particulate zinc oxide | | | | | | | | 9 |
| Colorant | Hydrophobically-treated* colorant | 1.75 | 1.75 | 1.75 | 1.75 | 1.75 | 1.75 | 1.75 | 1.75 |
| Water | | Balance | Balance | Balance | Balance | Balance | Balance | Balance | Balance |
| | | 100 | 100 | 100 | 100 | 100 | 100 | 100 | 100 |
| Stability | | A | A | A | B | A | B | A | A |
| Feeling in use | | A | A | B | A | B | A | B | B |
| Durability of makeup | | A | B | A | A | A | B | A | A |

| | | | | | | | | | |
|---|---|---|---|---|---|---|---|---|---|
| * Silicone treatment | | | | | | | | | |

**[Table 5-2]**

| | Raw materials | Comparative Example 5-1 | Comparative Example 5-2 | Comparative Example 5-3 | Comparative Example 5-4 | Comparative Example 5-5 | Comparative Example 5-6 |
|---|---|---|---|---|---|---|---|
| Core-corona type microgel | (Acrylates/methoxy PEG-90 methacrylate) crosspolymer | - | 1 | 1 | 3.5 | 0.25 | 12 |
| Agar microgel | Agar | 0.3 | - | 5.5 | 0.3 | 0.3 | 0.3 |
| Activator | PEG-60 hydrogenated castor oil | 1 | - | - | 0.5 | - | - |
| Alcohol | Ethanol | 1.5 | 1.5 | 1.5 | 1.5 | 1.5 | 1.5 |
| Moisturizer | Dipropylene glycol | 9 | 9 | 9 | 9 | 9 | 9 |
| | Glycerin | 2 | 2 | 2 | 2 | 2 | 2 |
| Preservative | Phenoxyethanol | 0.5 | 0.5 | 0.5 | 0.5 | 05 | 0.5 |
| Silicone oil | Decamethyl cyclopenta siloxane | 16 | 16 | 16 | 16 | 16 | 16 |
| Ultraviolet absorbent | Octylmethoxy cinnamate | 4 | 4 | 4 | 4 | 4 | 4 |
| Dispersant | Bis-Butyldimethicone Polyglyceryl-3 | 1.5 | 1.5 | 1.5 | 1.5 | 15 | 1.5 |
| | Isostearic acid | 1 | 1 | 1 | 1 | 1 | 1 |
| Hydrophobic powder | Hydrophobically-treated* pigment grade titanium oxide | 8 | 8 | 8 | 8 | 8 | 8 |
| | Hydrophobically-treated* particulate titanium oxide | 2.5 | 2.5 | 2.5 | 2.5 | 2.5 | 2.5 |
| Colorant Hydrophobically-treated* colorant | | 1.75 | 1.75 | 1.75 | 1.75 | 1.75 | 1.75 |
| Water | | Balance | Balance | Balance | Balance | Balance | Balance |
| | | 100 | 100 | 100 | 100 | 100 | 100 |
| Stability | | A | C | A | A | C | A |
| Feeling in use | | D | A | C | C | A | C |
| Durability of makeup | | D | A | A | A | C | A |

| | | | | | | | |
|---|---|---|---|---|---|---|---|
| * Silicone treatment | | | | | | | |

Table 5 shows that the effect can be achieved when the hydrophobically-treated powder is blended to the internal oil phase.

It is obvious from Test examples 5-1, 5-4 and 5-5 and Comparative examples 5-2 and 5-3 that that when the blending amount of the agar microgel is too small, stability deteriorates and when too much, freshness deteriorates. In addition, it is obvious from Test examples 5-1, 5-6 and 5-7 and Comparative examples 5-5 and 5-6 that when the amount of the core-corona type microgel is too small, emulsification becomes poor and stability thereof is impaired and when too much, freshness deteriorates.

The emulsifiers can be blended in a range of not affecting usage of the cosmetic as shown in Test examples 5-1, 5-2, 5-3 and 5-8 and Comparative example 5-4.

It was found that the higher the blending amount of the core-corona type microgel is, the better the durability of makeup after 4 hours and the suppression of the occurrence of irregularity on the makeup are as shown in Tables 4 and 5.

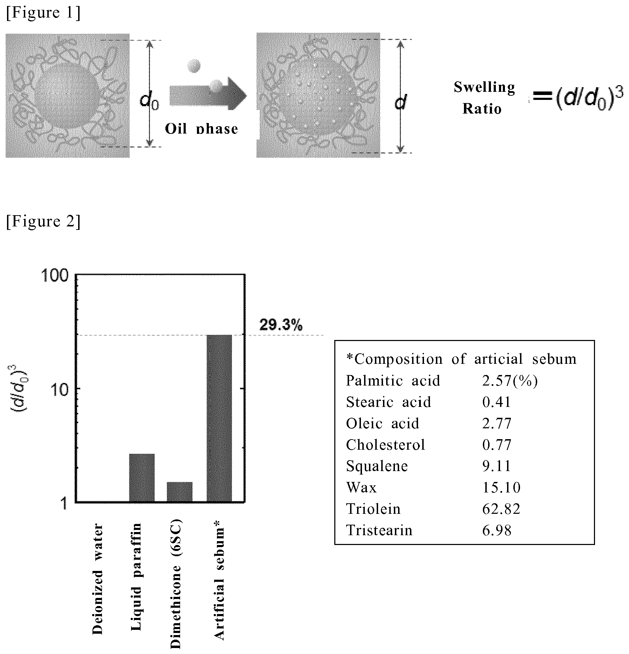

The present inventors measured the swelling ratio (d/d₀)³ of the core-corona type microgel with respect to each oil component (see Figure 1) to elucidate the above phenomenon. The results are shown in Figure 2.

As shown in Figure 2, it was found that although the swelling ratio between liquid paraffin and dimethicone is not more than 10%, the swelling ratio of the artificial sebum is 29.3%, which is extremely high. The fact that the swelling ratio of the artificial sebum is higher than the swelling ratio between liquid paraffin and dimethicone indicates that the core-corona type microgel enables the sebum secreted on skin to be more selectively absorbed, so that it is obvious that the experiment results in Tables 4 and 5 are supported.

Formulation examples are shown in the following, but the present invention is not limited thereto.

**Formulation example 1: Foundation**

| | |
|---|---|
| (Acrylates/methoxy PEG-90 methacrylate) crosspolymer | 1% |
| Agar | 0.3% |
| Ethanol | 1.5% |
| Dipropylene glycol | 9% |
| Dimethicone | 12.5% |
| Octyl methoxycinnamate | 7.5% |
| Amodimethicone | 2% |
| Silicone-treated pigment grade titanium oxide | 5% |
| Silicone-treated particulate titanium oxide | 3% |
| Silicone-treated iron oxide | 2% |
| Deionized water | Balance |

**Formulation example 2: Sunscreen**

| | |
|---|---|
| Polymer of Production example 5 | 1% |
| Agar | 0.5% |
| Ethanol | 1.5% |
| Dipropylene glycol | 9% |
| Dimethicone | 12.5% |
| Octyl methoxycinnamate | 7.5% |
| Amodimethicone | 2% |
| Silicone-treated particulate titanium oxide | 7% |
| Silicone-treated particulate zinc oxide | 5% |
| Deionized water | Balance |

**Formulation example 3: Body Cream**

| | |
|---|---|
| (Acrylates/methoxy PEG-90 methacrylate) crosspolymer | 1.5% |
| Agar | 1.5% |
| Ethanol | 0.5% |
| Dipropylene glycol | 9% |
| Glycerin | 3% |
| Isododecane | 7% |
| Triethylhexanoin | 5% |
| Liquid paraffin | 5% |
| Deionized water | Balance |

**Formulation example 4: Sunscreen Cosmetic**

| | |
|---|---|
| (Acrylate/alkyl acrylate (C10-30)) crosspolymer | 0.1 |
| Lauryl betaine | 1.0 |
| Diisopropyl sebacate | 7.0 |
| Dimethicone | 3.0 |
| (Acrylates/methoxy PEG-90 methacrylate) crosspolymer | 1.0 |
| Carbomer | 0.16 |
| Agar | 0.1 |
| Xanthan gum | 0.1 |
| PPG-17/PPG-17/PPG-17 | 1.0 |
| Hydrophobilized titanium oxide | 4 |
| Ethylhexyl Methoxycinnamate | 6.0 |
| Ethylhexyl Triazone | 1.0 |
| Bis-ethylhexyloxyphenol methoxyphenyl triazine | 2.0 |
| Diethylamino hydroxybenzoyl hexyl benzoate | 2.0 |
| Phenoxyethanol | 0.5 |
| Alcohol | 5.0 |
| Potassium hydroxide | Suitable amount |
| Purified water | Balance |
| Perfume | Suitable amount |

## Claims

1. An oil-in-water emulsion composition, comprising:
an oil phase;
an aqueous phase in which said oil phase is dispersed;
a core-corona microgel in which a hydrophilic group is partially provided on a surface of a hydrophobic gel fine particle as a dispersant that disperses said oil phase in said aqueous phase; and
an agar microgel having an average particle size of 10 to 100 µm.

2. The oil-in-water emulsion composition according to claim 1, wherein 0.5 to 10% by mass of an (acrylates/methoxy PEG methacrylate) crosspolymer is comprised as said core-corona microgel.

3. The oil-in-water emulsion composition according to claim 1, wherein 0.5 to 10% by mass of an (acrylamide/acrylates/methoxy PEG methacrylate) crosspolymer is comprised as said core-corona microgel.

4. The oil-in-water emulsion composition according to any one of claims 1 to 3, wherein a powder is dispersed inside an internal oil phase.

5. The oil-in-water emulsion composition according to any one of claims 1 to 4, wherein a blending amount of a non-ionic surfactant is not more than 3% by mass in the composition.

6. The oil-in-water emulsion composition according to any one of claims 1 to 5, wherein said viscosity of said composition is not higher than 50,000 mPa·s.

7. The oil-in-water emulsion composition according to any one of claims 1 to 6, wherein said viscosity of said composition is not higher than 10,000 mPa·s.

8. The oil-in-water emulsion composition according to any one of claims 1 to 7, wherein said viscosity of said composition is not higher than 5,000 mPa·s.
